# EUROPEAN PATENT APPLICATION

(11) **EP 2 018 846 A1**
(43) Date of publication of application: **28.01.2009**
(21) Application number: 08013063.6
(22) Date of filing: 19.07.2008
(51) Int. Cl.: A61K 8/41, A61Q 5/06

(54) **Process for colouring keratin fibres**

(30) Priority: 27.07.2007 EP 07014732
(71) Applicant: KPSS Kao Professional Salon Services GmbH, 64297 Darmstadt (DE)
(72) Inventor: Petzke, Erika, 64319 Pfungstadt (DE)
(74) Representative: Grit, Mustafa

(57) **Abstract**

This invention relates to a process for colouring keratin fibres especially human hair. Accordingly, the object of the present invention is a process for colouring keratin fibres, especially human hair, wherein keratin fibres to be coloured is wetted with water before application of a colouring composition comprising at least one direct dyestuff dispersed in a suitable medium for colouring wherein part of direct dyestuff is present in undissolved form.

## Description

This invention relates to a process for colouring keratin fibres especially human hair.

Colouring human hair is a common practice since many decades. Colouring is done in two different was namely oxidative colouration which delivers intensive and long lasting hair colours and colouring with direct dyes which delivers also intensive colours but the colours wash out relatively easily since in most of the cases the dyeing mechanism is dyestuff adsorption onto hair surface rather than dyestuff penetration into the fibres.

Furthermore, especially with the direct dyes, homogeneous colouration of keratin fibres especially hair is important. Adsorption and/or penetration of direct dyes onto/into keratin fibres varies strongly as a function of hair damage, i.e. uptake onto/into highly damaged hair is high. Another point to mention is the dyestuff concentrations used in the formulations is relatively high and that result in partly presence of partly undissolved dyestuff. Presence of undissolved dyestuff particles is especially high when carrier composition comprises high level of lipophilic material especially oils of any type.

In case colouring mass includes undissolved dispersed dyestuff particles and especially in a lipophilic medium, it has been observed that with the conventional dyeing process homogeneous coloration is not possible. Hair colour is very inhomogeneous and this is also not very much dependent upon the physical status of hair.

The present invention starts from the problems mentioned above and provides a process where homogeneous colours are obtained.

Accordingly, the first object of the present invention is a process for colouring keratin fibres, especially human hair, wherein keratin fibres to be coloured is wetted with water before application of a colouring composition comprising at least one direct dyestuff dispersed in a suitable medium for colouring wherein part of direct dyestuff is present in undissolved form.

Further object of the present invention is the use of the above process for homogeneous colouring hair.

The direct dyes suitable are in principal any direct dye known in the art for colouring hair. The direct dyes can be cationic, anionic and/or neutral one or their mixtures.

All cationic dyes available for hair colouration are suitable for the use in the colouring process of the present invention. Suitable examples are Basic Blue 6, Basic Blue 7, Basic Blue 9, Basic Blue 26, Basic Blue 41, Basic Blue 99, Basic Brown 4, Basic Brown 16, Basic Brown 17, Natural Brown 7, Basic Green 1, Basic Orange 31, Basic Red 2, Basic Red 12 Basic Red 22, Basic Red 51, Basic Red 76, Basic Violet 1, Basic Violet 2, Basic Violet 3, Basic Violet 10, Basic Violet 14, Basic Yellow 57 and Basic Yellow 87.

For the anionic dyes, again any anionic dye known in the art and suitable for colouring keratin fibres is in principal suitable for the use in the process of the present invention. Suitable examples are such as Acid Black 1, Acid Blue 1, Acid Blue 3, Food Blue 5, Acid Blue 7, Acid Blue 9, Acid Blue 74, Acid Orange 3, Acid Orange 6, Acid Orange 7, Acid Orange 10, Acid Red 1, Acid Red 14, Acid Red 18, Acid Red 27, Acid Red 50, Acid Red 52, Acid Red 73, Acid Red 87, Acid Red 88, Acid Red 92, Acid Red 155, Acid Red 180, Acid Violet 9, Acid Violet 43, Acid Violet 49, Acid Yellow 1, Acid Yellow 23, Acid Yellow 3, Food Yellow No. 8, D&C Brown No. 1, D&C Green No. 5, D&C Green No. 8, D&C Orange No. 4, D&C Orange No. 10, D&C Orange No. 11, D&C Red No. 21, D&C Red No. 27, D&C Red No. 33, D&C Violet 2, D&C Yellow No. 7, D&C Yellow No. 8, D&C Yellow No. 10, FD&C Red 2, FD&C Red 40, FD&C Red No. 4, FD&C Yellow No. 6, FD&C Blue 1, Food Black 1, Food Black 2, Disperse Black 9 and Disperse Violet 1 and their alkali metal salts such as sodium, potassium.

Among those, the preferred anionic dyestuffs are Acid Red 52, Acid Violet 2, Acid Red 33, Acid Orange 4, Acid Red 27 and Acid Yellow 10 and their salts. The most preferred anionic dyes are Acid Red 52, Acid Violet 2, Acid Red 33, Acid Orange 4 and Acid Yellow 10, and their salts

Any neutral nitro dye is also suitable for the purpose of the present invention. Non-limiting examples are Suitable ones are HC Blue No.2, HC Blue No.4, HC Blue No.5, HC Blue No.6, HC Blue No.7, HC Blue No.8, HC Blue No.9, HC Blue No.10, HC Blue No.11, HC Blue No.12, HC Blue No.13, HC Brown No.1, HC Brown No.2, HC Green No.1, HC Orange No.1, HC Orange No.2, HC Orange No.3, HC Orange No.5, HC Red BN, HC Red No.1, HC Red No.3, HC Red No.7, HC Red No.8, HC Red No.9, HC Red No.10, HC Red No.11, HC Red No.13, HC Red No.54, HC Red No.14, HC Violet BS, HC Violet No.1, HC Violet No.2, HC Yellow No.2, HC Yellow No.4, HC Yellow No.5, HC Yellow No.6, HC Yellow No.7, HC Yellow No.8, HC Yellow No.9, HC Yellow No.10, HC Yellow No.11, HC Yellow No.12, HC Yellow No.13, HC Yellow No.14, HC Yellow No.15, 2- Amino-6-chloro-4-nitrophenol, picramic acid, 1,2-Diamino-4-nitrobenzol, 1,4-Diamino-2-nitrobenzol, 3-Nitro-4-aminophenol, 1-Hydroxy-2-amino-3-nitrobenzol and 2-hydroxyethylpicramic acid.

It should be noted that the above mentioned dyes can also be used in mixture with each other without any limitation. In such a case compatibility between the dyes should be paid attention. The weight or molar ratio of dyes in mixture is not critical for the purpose of the present invention.

Concentration of any of the direct dye category or in total as a mixture is in the range of 0.01 to 10%, preferably 0.05 to 7.5%, more preferably 0.1 to 5% by weight calculated to total composition.

The medium wherein one or more direct dye is dispersed can be aqueous and preferably comprises at least 10%, preferably at least 20% by weight lipophilic compound which is liquid at the application temperature which may vary between room temperature, approximately 20°C, and 45°C.

The composition wherein at least one direct dye is dispersed is in principal aqueous and therefore comprises at least 5%, preferably 10% and more preferably 25% and most preferably 50% by weight water.

As a lipophilic compound any oil or oil like compounds are suitable. Non-limiting examples are mineral oil, silicone oils, faty acid alkyl esters, fatty acid glycerine esters wherein at least two acyl chain are present and triglycerides either natural oil or synthetic ones and di alkyl carbonates.

Example to silicone oil is dimethylpolysiloxanes or their derivatives. Silicone oils may be volatile or non-volatile, arylated or cyclic ones. Non-limiting examples are dimethicone, dimethiconol, cyclomethicone, cyclopentasiloxane, phenyl trimethicone, phenyl propyl trimethicone, pentaphenyltrisiloxane, and amodimethicone.

Suitable fatty acid alkyl esters are according to the formula

R₁ C (O) O R₂

wherein R1 is a saturated or unsaturated, straight or branched alkyl chain with 7 to 23 C atoms and R2 is a saturated or unsaturated, straight or branched alkyl chain with 8 to 24 C atoms.

Non-limiting examples are cetyl palmitate, isopropyl myristate, isopropyl palmitate, isopropyl stearate, decyl oleate, stearyl behenate, oleyl linoleate, oleyl myristate, oleyl oleate, octyldodecyl oleate, octyldodecyl cocoate, myristyl stearate, myristyl laurate and myristyl myristate.

Glycerides and especially triglycerides are known natural oils and/or synthetic ones. Non-limiting examples are almond oil, avocado oil, peach oil, wheatgerm oil, olive oil, sunflower oil and soya oil.

Suitable alkyl carbonates are those according to general formula

R₂ O C (O) O R₂

R₂ has the same meaning as above.

Suitable non-limiting examples are di ethylhexyl carbonate, dioctyl carbonate and dicaprylyl carbonate.

Preferred are triglycerides, fatty acid alkyl esters of any type, silicone oils either volatile or non-volatile and mineral oil such as paraffin oil.

In the process of the present invention it is also possible to disperse the dyestuff powder prior to application onto hair with a composition as disclosed above.

Accordingly, further object of the present invention is process for colouring keratin fibres wherein at least one direct dye is dispersed prior to application into a suitable medium and applied onto wet hair.

In the above process the suitable medium comprises at least 10% by weight lipophilic compound and at least 10% by weight water.

In a further preferred form of the present invention, dispersed direct dye composition is further mixed with a composition comprising at least one oxidizing agent. Any oxidizing agent known in the art is suitable as a rule, however most preferred is hydrogen peroxide and at a concentration of 2 to 12% by weight calculated to total composition prior to mixing.

pH of the compositions comprising dispersed direct dye is in the range of 2 to 12. Especially when composition comprising dispersed direct dye is mixed with a composition comprising at least one oxidizing agent, than pH is preferably 5 to 12, more preferably, 6 to 10.5 and most preferably 8 to 10.

Compositions used in the process of the present invention can be in the form of a thin liquid, composition with two distinctly separated layers, thickened liquids, emulsions and gels. In order to prepare such compositions required additional ingredient may be used wherever appropriate and necessary.

The composition can comprise one or more surfactants selected from anionic, nonionic, cationic and amphoteric ones. Their proportion ranges from 0.05 % to 10%, in particular from 0.1 % to 5 % by weight, calculated to total composition.

Suitable anionic surfactants are especially the known alkyl ether sulfates and carboxylic acids, in particular in form of their alkali salts, as well as protein fatty acid condensates.

Suitable nonionic surfactants, which are preferred within the scope of the invention, are in particular C₈-C₁₈-fatty alcohol polyglycol ethers, fatty acid polyglycol esters, fatty acid alkanolamides, amineoxides, and especially C₈-C₁₈-alkyl polyglucosides.

Also possible is the incorporation of amphoteric surfactants, such as the known alkyl betaines, alkyl amido betaines, and alkyl amphoacetates.

Further according to a further preferred embodiment, the above mentioned compositions comprise at least one cationic surfactant according to general formula 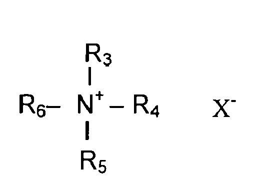 where R₃ is a saturated or unsaturated, branched or non-branched alkyl chain with 8-22 C atoms or

R₇ CO NH (CH₂)ₙ

where R₇ is saturated or unsaturated, branched or non-branched alkyl chain with 7-21 C atoms and n has typical value of 0 - 4 or

R₈ CO O (CH₂)ₙ

where R₈ is saturated or unsaturated, branched or non-branched alkyl chain with 7-21 C atoms and n has typical value of 0 - 4, and
R₄ is a hydrogen, saturated or unsaturated, branched or non-branched alkyl chain with 1-22 C atoms or

R₇ CO NH (CH₂)ₙ

where R₇ is saturated or unsaturated, branched or non-branched alkyl chain with 7-21 C atoms and n has typical value of 0 - 4 or

R₈ CO O (CH₂)ₙ

where R₈ is saturated or unsaturated, branched or non-branched alkyl chain with 7-21 C atoms and n has typical value of 0 - 4,
and R₅ and R₆ are independent from each other H or lower alkyl chain with 1 to 4 carbon atoms, and X is chloride, bromide or methosulfate.

Concentration of cationic surfactant is in the range from 0.05 % to 5 %, preferably 0.1 % to 2.5 % by weight, calculated to total composition.

Suitable long-chain quaternary ammonium compounds which can be used alone or in admixture are in particular cetyl trimethyl ammonium chloride, dimethyl dicetyl ammonium chloride, trimethyl cetyl ammonium bromide chloride, stearyl trimethyl ammonium chloride, dimethyl stearyl benzyl ammonium chloride, benzyl tetradecyl dimethyl ammonium chloride, dimethyl dihydrogenated tallow ammonium chloride, lauryl pyridinium chloride, lauryl dimethyl benzyl ammonium chloride, lauryl trimethyl ammonium chloride, tris-(oligooxyethyl) alkyl ammonium phosphate, cetyl pyridinium chloride, etc.

Additional examples to so called ester and amido quaternary ammonium compounds are distearyldimonium chloride, dipalmitoylethylhydroxyethylmonium chloride, dioleoylethyl dimethyl ammonium methosulfate, dioleoylethyl hydroxyethylmonium methosulfate, dilinolamidopropyldimonium chloride, dioleylethyl hydroxyethylmonium chloride, dipalmitoylethyldimonium chloride.

From the above quaternary ammonium compounds disclosed with the general formula, especially to mention are those compounds known per se and are on the market, for example, under the trade names "Schercoquat^{®}", "Dehyquart^{®} F30" and "Tetranyl^{®}". Use of these compounds, the so-called "esterquats", in hair care compositions is described, for example, in WO-A 93/107 48, WO-A 92/068 99 and WO-A 94/166 77, wherein, however, there is no reference made to the combinations according to the present invention and the advantageous properties thereof.

Again from the above quaternary ammonium compounds disclosed with the general formula, especially to mention are those compounds are known per se and on the market, for example, under the trade name "INCROQUAT^{®} HO" or "OCS". These compounds are known with a general ingredient category under "amidoquat" in the cosmetic industry.

Compositions mentioned above can also comprise thickening. All known thickening agents such as anionic, non-ionic, cationic polymers are suitable for the purpose of the invention. It should be noted that compatibility with various ingredients of individual compositions should be paid attention when selecting thickening polymer. Suitable ones are cellulose derivatives such as hydroxyethyl or methyl cellulose, anionic acrylate polymers, cationic cellulose derivatives.

Thickening of the compositions can also be achieved by formulating an emulsion. In such a case at least one fatty alcohol with an alkyl chain length of 12 to 22 C atoms should be present in the composition. Examples are cetyl alcohol, stearyl alcohol or their mixture, myristyl alcohol and behenyl alcohol. Branched fatty alcohols such as octyldodecanol may also be present either alone or in mixture with kinear fatty alcohols. Emulsions must also comprise an emulsifier selected from anionic, non-ionic and cationic surfactants as mentioned above. Most preferred emulsifies are those ethoxylated fatty alcohols as nonionc ones, alkyl sulfates or alkyl ether sulfates as anionc ones and monoalkyl quaternary ammonium ones as cationic surfactants.

Further, the above mentioned compositions may comprise additional cationic polymer. Basically suitable are all cationic polymers listed under the generic name "Polyquaternium" in the CTFA International Cosmetic Ingredient Dictionary. Examples are Polyquaternium 6, Polyquaternium 7, Polyquaternium 10, Polyquaternium 11, Polyquaternium 16, Polyquaternium 22 and Polyquaternium 28, and Polyquaternium 39.

The cationic polymers also include the quaternized products of graft polymers from organopolysiloxanes and polyethyl oxazolines described in EP-A 524 612 and EP-A 640 643. Such polymer is known with its CTFA name Polysilicone-9.

Concantration of one or more additional cationic polymers is in the range from 0.05 % to 2.5 %, preferably 0.1 % to 1.5 % by weight, calculated to total composition.

Further the above mentioned composition comprise preferably at least one organic solvent. Suitable organic solvents are 2-methyl-1,3-propanediol, mono and dialcohols or the ethers thereof, in particular mono-C₁-C₃-alkyl ether, ethanol, n-propanol, isopropyl alcohol, 1-methoxypropanol, 1-ethoxypropanol and ethoxydiglycol, diols and their esters 1,3- and 1,4-butanediol, diethyleneglycol and the monomethyl and monoethyl ether thereof, dipropylene glycol and the monomethyl and monoethyl ether thereof, glycerol, hexanetriol, ethyl carbitol, benzyl alcohol, benzyloxy ethanol, propylene carbonate, N-alkyl pyrrolidone, and urea or their mixture preferably in an amount from about 0.1 % to 10 % by weight, calculated to the total composition.

The above mentioned compositions can comprise further ceramide type of compound such as cetyl-PG-hydroxyethylpalmitamide.

Further optional ingredient are sterols, especially the phytosterols are useful hair restructuring compounds can be present in the above mentioned compositions. Especially preferred ones are of plant origin for example ergosterol, sitosterol, stigmasterol, fucosterol, brassicasterol, fungisterol, campesterol, zymosterol, ascosterol, cerevisterol, episterol, faecosterol, spinasterol. Among those phytosterols, the ones found in "Avocadin" which is the unsaponified fraction of the avocado oil is more preferred.

Optionally fatty acids of C10 to C22 may be incorporated into the compositions of the present invention at a concentration of preferably 0.01 to 10%, preferably 0.1 to 5 and more preferably 0.2 to 2.5% by weight calculated to total composition.

Further additional compounds may be present in the above mentioned compositions of the present invention is ubichinone of the formula 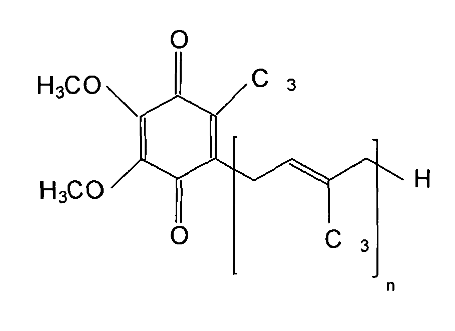 where n is a number between 1 and 10. Preferred ubichinones are the ones where n is a number between 6 and 10 and especially preferred is Ubichinone 50 where n is 10, also known as Coenzyme Q10. Concentration ubichinone of the above formula in the compositions is from 0.0001 to 1%, preferably from 0.0002 to 0.75%, more preferably from 0.0002 to 0.5% and most preferably from 0.0005 to 0.5% by weight, calculated to total composition.

Furthermore, compositions comprising at least one dispersed direct dye may comprise in principal at least one oxidative dye precursors and at least one coupling agent.

Suitable non-limiting examples to oxidative dye precursors are p-phenlynediamine, p-methylaminophenol and substituted p-phenylenediamines such as 2,5-diamino-toluene, 2-n-propyl or 2-ethyl-p-phenylenediamine, 2,6-di-methyl-p-phenylenediamine, 2-(2,5-diaminophenyl) ethanol, 1-amino-4-bis-(2'-hydroxy-ethyl)aminobenzene, 2-(2-hydroxyethyl amino)-5-aminotoluene, 4,4'-diaminodiphenylamine, 4-aminodiphenylamine, 2-amino-5-N,N-diethyl aminotoluene, 4-amino-N-ethyl-N-isopropyl aniline, 2-chloro-p-phenylenediamine, 1-β-hydroxyethyl-2,5-diamino-4-chlorobenzene, 1-β-hydroxyethyl-2,5-diamino-4-methyl benzene, 2-methoxy-p-phenylenediamine, N,N-diethyl-p-phenylenediamine, 1-amino-4-β-methoxyethyl aminobenzene, 1-dimethyl-amino-4-aminobenzene, 1-hydroxy-2,5-diamino-4-methyl benzene, 1-hydroxymethyl-2,5-diaminobenzene, 1,3-dimethyl-2,5-diaminobenzene, 1,4-diamino isopropyl benzene and/or 1-amino-4-β-hydroxypropyl aminobenzene, pyrazole and the derivatives thereof such as 1-hydroxyethyl-4,5-diaminopyrazole, 3,4-diamino-5-hydroxypyrazole, 3,5-diaminopyrazole, 3,5-diamino pyrazol-1-carboxamide, 3-amino-5-hydroxypyrazole, 1-phenyl-2-methylpyrazole, 1-phenyl-3-methylpyrazole-5-one, 3,5-dimethylpyrazole, 3,5-dimethylpyrazole-1-methanol, 3,5-diamino-1,2,4-triazole, 4-aminophenol and the derivatives thereof such as 4-amino-3-methylphenol, 2-chloro-4-aminophenol, 2,6-dichloro-4-aminophenol, 2,4-diaminophenol, 2,6-dibromo-4-aminophenol, tetraamino pyrimidines, triaminohydroxy pyrimidines, diaminomono- and -dihydroxy pyrimidines, aminotriazines, 5-amino salicylic acid and/or 1,2,4-triamino benzene or the water-soluble salts thereof.

Further suitable aminopyridines are 2,5-diaminopyridine, 2,3-diaminopyridine, 2,6-diaminopyridine, 3-amino-2-methyl amino-6-methoxypyridine, 2-dimethyl-5-aminopyridine, 2-dimethyl aminoethyl-3-hydroxypyridine, 2-amino-4,6-dimethyl pyridine, 2-amino-3-hydroxypyridine, 3-amino-2(β-hydroxyethyl amino)-6-methoxy-pyridine, 2,6-dimethyl amino-5-aminopyridine, 2-di(hydroxyethyl) amino-5-aminopyridine, 2-hydroxyethyl amino-5-aminopyridine, 4-hydroxy-2,5,6-triaminopyrimidine and/or the water-soluble salts thereof.

Further, Indole and indoline derivatives can as well be contained in the colouring composition of the present invention. Examples to those are: 6-aminoindol, 6-hydroxyindole, 1-ethyl-6-hydroxyindole, 1-methyl-4-hydroxyindol, 1-methyl-6-hydroxyindole, 2-methyl-6-hydroxyindole, 5-hydroxyindol, 4-hydroxyindol, 5,6-dihydroxyindole, 6-aminoindoline, 6-hydroxyindoline, 1-ethyl-6-hydroxyindoline, 1-methyl-4-hydroxyindoline, 1-methyl-6-hydroxyindoline, 2-methyl-6-hydroxyindoline, 5-hydroxyindoline, 4-hydroxyindoline, 5,6-dihydroxyindoline and their respective salts.

Non-limiting examples to suitable coupling agents are resorcinol, 2-methyl resorcinol, 4-chlororesorcinol, 2-amino-4-chlorophenol, 5-amino-4-methoxy-2-methylphenol, 3-amino-phenol, 1-methyl-2-hydroxy-4-aminobenzene, 3-N,N-dimethyl aminophenol, 2.6-dihydroxy-3.5-dimethoxypyridine, 5-amino-3-methylphenol, 6-amino-3-methylphenol, 3-amino-2-methylamino-6-methoxypyridine, 2-amino-3-hydroxypyridine, 2-dimethyl-amino-5-aminopyridine, 2,6-diaminopyridine, 1,3-diaminobenzene, 1-amino-3-(2'-hy-droxyethylamino)benzene, 1-amino-3-[bis(2'-hydroxyethyl) amino]benzene, α-naphthol, 4,6-dichlororesorcinol, 1,3-diamino-toluene, 1-hydroxy naphthalene, 4-hydroxy-1,2-methylenedioxy benzene, 1,5-dihydroxy naphthalene, 1,6-dihydroxy naphthalene, 1,7-dihydroxy naphthalene, 2,7-dihydroxy naphthalene, 1-hydroxy-2-methyl naphthalene, 4-hydroxy-1.2-methyldioxy benzene, 2,4-diamino-3-chlorophenol, 5-amino-2-methoxyphenol and/or 1-methoxy-2-amino-4-(2'-hydroxyethyl amino)benzene or the water-soluble salts thereof. 5-amino-2-methylphenol, 2-methyl-5-hydroxyethylaminophenol, 2,4,-diamnophenoxyehanol, 2-amino-4-hydroxyethylaminoanisol, 2-methyl-5-amino-6-chlorphenol, 1,3-bis(2,4-diaminophenoxy)propane, 2-bis(2-hydroxyethyl)aminotoluene, 2-amino-5-methylphenol or the water-soluble salts thereof.

Concentration of oxidation dyes precursors and couplers is customarily in the range of 0.01 to 5% by weight calculated to total composition prior to mixing with oxidizing agent.

Further, with the novel process of the present invention, any type of keratin fibres may be coloured intensive and homogeneous. Hair tresses may undergone a previous chemical processing such as permanent shaping or bleaching or oxidative colouration.

Following example should illustrate the invention but not limit it.

### Example 1

**Colouring composition**

| | % by weight |
|---|---|
| Basic red 51 | 0.5 |
| Mineral oil | 89.5 |
| Water | 10.0 |

Using the above composition two series of bleached hair tresses were coloured. For comparative purposes one of the tress was dry and the other tress was wetted by water and towel dried.

After colouring the tresses were dried and L, a, and b values were measured using a laboratory chromometre. The results obtained are presented in Table I.

**Tablel: Results of L,a, b value measurements**

| | | Wet hair | | | | Dry hair | |
|---|---|---|---|---|---|---|---|
| Measurement | | | | | | | |
| 1 | 31.78 | 23.68 | 8,91 | | 31.67 | 18.28 | 15.06 |
| 2 | 31.48 | 23.48 | 9,36 | | 31.80 | 22.16 | 14.55 |
| 3 | 31.26 | 23.75 | 10,02 | | 32.66 | 23.32 | 15.74 |
| 4 | 31.33 | 23.98 | 9,49 | | 30.78 | 24.92 | 14.99 |
| 5 | 30.98 | 23.13 | 10,38 | | 31.38 | 27.33 | 14.72 |
| 6 | 32.12 | 23.42 | 10,23 | | 31.01 | 27.90 | 13.37 |
| 7 | 31.75 | 23.38 | 9,99 | | 28.29 | 30.27 | 11.89 |
| 8 | 31.13 | 23.91 | 9,72 | | 28.11 | 29.52 | 11.44 |
| 9 | 31.49 | 24.02 | 10,09 | | 32.39 | 20.23 | 15.79 |
| 10 | 31.65 | 23.56 | 9,86 | | 33.57 | 17.96 | 15.88 |
| | | | | | | | |
| Avarage | 31.50 | 23.63 | 14.72 | | 31.17 | 28.19 | 14.34 |
| Standard deviation | 0.34 | 0.29 | 0.43 | | 1.76 | 4.52 | 1.56 |

From the above results it is clear that hair tress coloured according to the novel inventive process, is homogeneous and slightly more intensive. This is clear as all standard deviation values are smaller that the comparative one.

## Claims

1. Process for colouring keratin fibres, especially human hair, **characterised in that** keratin fibres to be coloured is wetted with water before application of a colouring composition comprising at least one direct dyestuff dispersed in a suitable medium for colouring wherein part of direct dyestuff is present in undissolved form.

2. Process according to claim 1 **characterised in that** at least direct dye is chosen from anionic, cationic and neutral nitro dyestuffs at a concentration in the range of 0.01 to 10% by weight calculated to total composition.

3. Process according to any of the preceding claims **characterised in that** it comprises at least 10% by weight, calculated to total composition, lipophilic compound and at least 5% by weight, calculated to total composition, water.

4. Process according to claim 3 **characterised in that** lipophilic compound is a volatile or non-volatile silicone oil.

5. Process according to claims 3 and 4 **characterised in that** lipophilic compound is either natural or synthetic natural oil.

6. Process according to claims 3 to 5 **characterised in that** lipophilic compound is a fatty acid alkyl ester according to the formula
R₁ C (O) O R₂
wherein R₁ is a saturated or unsaturated, straight or branched alkyl chain with 7 to 23 C atoms and R₂ is a saturated or unsaturated, straight or branched alkyl chain with 8 to 24 C atoms.

7. Process according to claims 3 to 6 **characterised in that** lipophilic compound is an alkyl carbonate according to general formula
R₂ O C (O) O R₂
wherein R₂ is a saturated or unsaturated, straight or branched alkyl chain with 8 to 24 C atoms.

8. Process according to any of the preceding claims **characterised in that** at least one direct dye powder is mixed prior to application onto hair.

9. Process according to any of the preceding claims **characterised in that** the composition comprising at least one dispersed direct dye is mixed with a composition comprising at least one oxidizing agent at a concentration of 2 to 12% by weight, calculated to total composition prior to mixing.

10. Process according to any of the preceding claims **characterised in that** ph of the composition wherein at least one direct dye is dispersed and present partly in an undissolved form has a pH between 2 and 12.

11. Process according to any of the preceding claims **characterised in that** it comprises at least one surfactant selected from anionic, non-ionic, cationic and amphoteric ones at a concentration of 0.05 to 10% by weight calculated to total composition.

12. Process according to any of the preceding claims **characterised in that** it comprises at least one cationic polymer and/or at least one thickening polymer and/or at least one fatty alcohol with an alkyl chain length of 12 to 22 C atoms and/or at least one organic solvent and/or at least one compound according to general formula 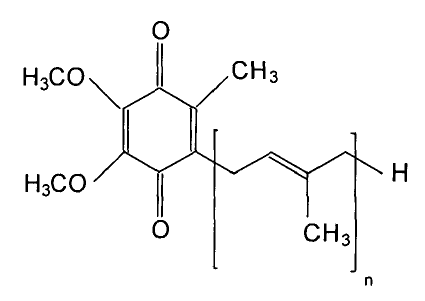 wherein n is a number between 1 and 10

13. Process according to any of the preceding claims **characterised in that** it comprises at least one oxidative dye precursor and at least one coupler.

14. Use of the process according to claims 1 to 18 for homogeneous and intensive colouring hair.

15. Use of the process according to claims 1 to 18 for homogeneous and intensive colouring bleached, permanently shaped and oxidative coloured hair.
